# EUROPÄISCHE PATENTSCHRIFT

(11) **EP 2 277 598 B1**
(45) Veröffentlichungstag und Bekanntmachung des Hinweises auf die Patenterteilung: **02.08.2017**
(21) Anmeldenummer: 10177134.3
(22) Anmeldetag: 19.03.2007
(51) Int. Cl.: A61Q 5/08, A61K 8/46, A61K 8/49, A61K 8/86, D21H 21/32, D06L 4/30, D06P 1/62, D06P 5/13, D06P 5/15

(54) **Reduktiver Farbabzug**
Reductive colour removal
Décoloration réductive

(30) Priorität: 11.05.2006 DE 102006022214; 21.03.2006 DE 102006013260
(43) Veröffentlichungstag der Anmeldung: 26.01.2011
(62) Teilanmeldung aus: 07723364.1
(73) Patentinhaber: Henkel AG & Co. KGaA, 40589 Düsseldorf (DE)
(72) Erfinder: Müller, Burkhard, 21075, Hamburg (DE); Neubueser, Inge, 22587, Hamburg (DE); Groß, Wibke, 41836, Hückelhoven (DE)

(56) Entgegenhaltungen:
- FR-A1- 2 814 948
- US-A- 3 892 845

## Beschreibung

Die Erfindung betrifft Zusammensetzungen zum reduktiven Farbabzug, welche neben organischen Sulfinsäurederivaten mindestens ein nichtionisches Tensid enthalten. Diese Farbabzugsmittel eignen sich für die Entfärbung gefärbter Substrate wie Papier, Textilien und insbesondere keratinhaltigen Fasern, beispielsweise menschlicher Haare. Gleichfalls ist ein Verfahren zur Entfärbung gefärbter Substrate, sowie die Verwendung besagter Mittel zum Farbabzug Gegenstand der Erfindung.

Beim Färben wird der Farbstoff auf das Substrat durch Adsorption an die Oberfläche, durch Eindiffundieren, durch Bildung auf und/oder in dem Substrat bzw. durch chemische Bindung übertragen. Für das Färben, beispielsweise von Papier, Textilien oder keratinhaltigen Fasern, kommen im allgemeinen entweder direktziehende Farbstoffe oder Oxidationsfarbstoffe in Frage. Oxidationsfarbstoffe entstehen durch oxidative Kupplung einer oder mehrerer Entwicklerkomponenten untereinander oder mit einer oder mehreren Kupplerkomponenten. Kuppler-und Entwicklerkomponenten werden auch als Oxidationsfarbstoffvorprodukte bezeichnet. Die oxidative Kupplung findet bevorzugt während der Färbevorgangs statt, damit die Farbstoffvorprodukte in das Substrat hinein diffundieren können und der Farbstoff sich in dem Substrat bildet. Durch die Größe des entstandenen Farbstoffmoleküls wird ein Auswaschen aus dem Substrat erschwert.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt. Als Kupplerkomponenten werden in der Regel m-Phenylendiaminderivate, Naphthole, Resorcin und Resorcinderivate, Pyrazolone, m-Aminophenole und substituierte Pyridinderivate verwendet.

Unter direktziehenden Farbstoffen werden im allgemeinen Farbstoffe verstanden, die bereits vor Beginn des Färbens vorgebildet sind und auf das Substrat aufziehen. Wichtige Vertreter dieser Farbstoffklasse sind beispielsweise Triphenylmethanfarbstoffe, Azofarbstoffe, Anthrachinonfarbstoffe oder Nitrobenzolfarbstoffe, welche jeweils kationische oder anionische Gruppen tragen können.

Ein wichtiges technisches Gebiet stellt neben dem Färben das Abziehen von Farbstoffen dar. Darunter versteht man im Allgemeinen das Entfernen von Färbungen oder Bedruckungen durch Auswaschen, chemische Veränderung oder Zerstörung des Farbstoffes. Die oxidative oder die reduktive Entfärbung gefärbter Materialien findet insbesondere bei der Entfärbung von Textilien oder Haaren Anwendung.

Eine oxidative Entfärbung führt meist zu guten Ergebnissen. Jedoch kann durch die starke Oxidationswirkung des zur Entfärbung benutzten Oxidationsmittels die Struktur des Substrats chemisch verändert werden. Dies geht mit einer unerwünschten physikalischen Änderung des Substrats einher. Textilien oder Haare können beispielsweise spröde werden oder insbesondere bei mehrmaliger Entfärbung gar brechen. Der visuelle Eindruck, die Haptik aber auch die Haltbarkeit des Substrats werden dadurch negativ beeinflusst.

Weniger Einfluss auf die Substratstruktur, insbesondere auf die Struktur keratinhaltiger Fasern, nehmen reduktive Entfärbemittel. Die reduktiven Entfärbemittel entfärben kaum die Naturhaarfarbe, sondern wirken lediglich auf die durch Färbung aufgetragenen Farbstoffe reduktiv ein. Es tritt somit kaum eine Aufhellung des Haars auf. Allerdings bedürfen die reduktiven Entfärbemittel einer Verbesserung der Entfärbeleistung.

Überwiegend nutzt man reduktiv wirkende Schwefelverbindungen zur Entfärbung. Bekanntermaßen eignen sich Dithionite oder Derivate der 1-Hydroxymethylsulfinsäure bzw. der 1-Aminomethylsulfinsäure als Reduktionsmittel.

Aus der Druckschrift US-A-3 892 845 sind dem Fachmann reduktive Farbabzugsmittel bekannt, mit deren Hilfe sich gefärbte keratinhaltige Fasern entfärben lassen. Als Reduktionsmittel ist 1-Hydroxymethylsulfinsäure oder ein Salz davon in den Entfärbemitteln enthalten. Gemäß DE-OS-1 617 829 lässt sich durch Zugabe von 1-Hydroxymethylsulfinsäure zu oxidativen Entfärbemitteln, enthaltend Wasserstoffperoxid und Persulfate, deren Bleichwirkung verstärken.

Laut Offenbarung der EP-A-1 079 018 eignen sich Aminoalkansulfinate der Formel R¹_{3-z}N (CR²R³-SO₂-M)_{z} (R², R³ = Wasserstoff oder (C₁ bis C₄)-Alkyl) zur teilweisen Entfärbung von mit Küpenfarbstoffen oder Schwefelfarbstoffen gefärbten oder bedruckten Textilien.

Gemäß WO-A1-99/18067 eignen sich 1-Hydroxyalkylsulfinsäuren bzw. 1-Aminoalkylsulfinsäuren, welche eine Carboxygruppe, eine Sulfonsäuregruppe, eine Acylgruppe, eine Aminocarbonylgruppe oder eine Alkoxycarbonylgruppe tragen, zur Entfärbung gefärbter Substrate. Besagte Derivate besitzen eine vergleichbare oder bessere Entfärbekraft wie die 1-Hydroxymethylsulfinsäure (*vide supra),* spalten jedoch kein Formaldehyd ab. In der WO-A1-02/30369 wird die Entdeckung beschrieben, dass sich mit den Sulfinsäure-Derivaten der WO-A1-99/18067 auch keratinhaltige Fasern, wie beispielsweise Haare, entfärben lassen. Weitere zur Entfärbung keratinhaltiger Fasern geeignete Sulfinsäurederivate finden sich in den Druckschriften WO-A1-03/026597 und WO-A1-03/41668.

Substrate, die mit den Sulfinsäurederivaten des Standes der Technik reduktiv entfärbt wurden, erfahren einige Zeit nach dem Farbabzug eine unerwünschte Nachdunkelung.

Aufgabe der vorliegenden Erfindung ist es, reduktive Entfärbemittel bereitzustellen, die das Substrat dauerhaft ohne Nachdunkelung entfärben. Die Substratstruktur soll dabei geschont werden. Ferner sollten die in den Entfärbemitteln eingesetzten Reduktionsmittel für eine kosmetische Verwendung physiologisch verträglich und toxikologisch unbedenklich sein.

Überraschenderweise wird die Aufgabe durch Mittel gelöst, die eine Kombination mindestens eines der erfindungsgemäßen organischen Sulfinsäurederivate mit mindestens einem nichtionischen Tensid enthalten. Die Entfärbung gefärbter Substrate, wie beispielsweise Papier, Textilien oder keratinhaltigen Fasern, insbesondere menschlichem Haar, gelingt verbessert, ohne dass sich nach dem Entfärbevorgang eine Nachdunkelung in dem hohen Maße einstellt, wie sie durch die Verwendung der Sulfinsäurederivate allein hervorgerufen wird. Die erfindungsgemäßen Mittel eignen sich besonders zur faserschonenden Entfärbung keratinhaltiger Fasern.

Unter keratinhaltigen Fasern sind beispielsweise Wolle, Pelze, Federn und insbesondere menschliche Haare zu verstehen.

Ein erster Gegenstand der Erfindung sind daher Mittel, die in einem Träger mindestens ein Sulfinsäurederivat der Formel (I) 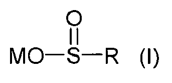 worin
- M: steht für ein Wasserstoffatom oder ein Äquivalent eines ein- oder mehrwertigen Kations,
- R: für einen Rest gemäß einer der Formeln (II) bis (III) steht, 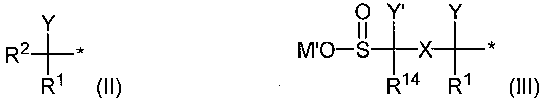 worin bedeuten
Y und Y' unabhängig voneinander eine Hydroxygruppe, eine -NH₂ Gruppe oder eine Gruppe -NR³R⁴, wobei R³ und R⁴ unabhängig voneinander für eine (C₁ bis C₆)-Alkylgruppe, eine (C₂ bis C₆)-Alkenylgruppe, eine (C₁ bis C₆)-Hydroxyalkylgruppe, eine (C₂ bis C₆)-Polyhydroxyalkylgruppe, eine Arylgruppe oder eine Aryl-(C₁ bis C₆)-alkylgruppe stehen,
M' unabhängig von M die unter M aufgeführten Merkmale,
X eine direkte Bindung oder einen organischen Rest mit zwei freien Valenzen,
R¹ und R¹⁴ unabhängig voneinander ein Wasserstoffatom, eine (C₁ bis C₆)-Alkylgruppe oder eine Carboxyalkylgruppe -(CH₂)ₘ-COOM^{II}, in der M" für ein Wasserstoffatom oder für ein Äquivalent eines ein- oder mehrwertigen Kations steht und m die Zahl 0, 1 oder 2 bedeutet,
R² ein Wasserstoffatom, eine (C₁ bis C₆)-Alkylgruppe, einen Carboxyalkylrest -(CH₂)ₙ-COOM^{III} mit
M^{III} = Wasserstoffatom oder ein Äquivalent eines ein- oder mehrwertigen Kations und n eine ganze Zahl 0, 1, 2, 3, 4, 5 oder 6,
einen (C₁ bis C₆)-Alkyloxycarbonylrest, eine N,N,N-Tri[(C₁ bis C₆)-alkyl]ammonium-(C₁ bis C₆)-alkylgruppe eine Carboxy-(C₂ bis C₆)-alkenylgruppe, eine Cyano-(C₁ bis C₆)-alkylgruppe oder eine (C₁ bis C₆)-Alkoxycarbonyl-(C₁ bis C₆)-alkylgruppe, oder
R² zusammen mit R¹ und dem Restmolekül einen aliphatischen 5-, 6-, oder 7-gliedrigen Ring bildet, welcher mindestens ein kationisches, quaterniertes Stickstoffatom als Heteroatom enthält, wobei die kationische Ladung gegebenenfalls durch ein Äquivalent eines ein- oder mehrwertigen Anions kompensiert wird, oder
einen aliphatischen oder aromatischen Heterozyklus, der substituiert sein kann,oder
einen Rest gemäß Formel (IV) 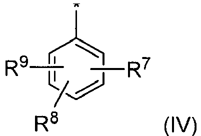 worin
R⁷ eine Carboxygruppe, eine Sulfonsäuregruppe, eine (C₁ bis C₆)-Alkoxycarbonylgruppe, eine Sulfonamidgruppe, eine Cyanogruppe, eine Nitrogruppe, eine Carboxy-(C₁ bis C₆)-alkylgruppe, eine Carboxy-(C₁ bis C₆)-alkoxygruppe oder eine Gruppe -N⁺R^{I}R^{II}R^{III}, mit R^{I}, R^{II} und R^{III} stehen unabhängig voneinander für eine (C₁ bis C₆)-Alkylgruppe, eine (C₂ bis C₆)-Alkenylgruppe, eine Aryl(C₁ bis C₆)-alkylgruppe oder eine (C₁ bis C₆)-Hydroxyalkylgruppe bedeutet,
R⁸ und R⁹ unabhängig voneinander ein Wasserstoffatom, eine (C₁ bis C₆)-Alkylgruppe, eine (C₂ bis C₆)-Alkenylgruppe, eine (C₁ bis C₆)-Hydroxyalkylgruppe, eine (C₂ bis C₆)-Polyhydroxyalkylgruppe, eine (C₁ bis C₆)-Alkoxygruppe, eine Hydroxygruppe, eine Aminogruppe, eine Carboxygruppe, eine Nitrogruppe, eine Cyanogruppe oder ein Halogenatom bedeuten,
mit der Maßgabe, dass in Formel (II) R¹ und R² nicht gleichzeitig für ein Wasserstoffatom stehen, in Kombination mit mindestens einem nichtionischen Tensid, wobei das nichtionische Tensid ausgewählt wird aus mindestens einem Vertreter der Gruppe, die gebildet wird, aus Anlagerungsprodukten von 2 bis 30 Mol Ethylenoxid und/oder 0 bis 5 Mol Propylenoxid an lineare Fettalkohole mit 8 bis 22 C-Atomen, enthalten.

Falls die Verbindungen gemäß Formel (I) mindestens ein Chiralitätszentum enthalten, sind selbstverständlich alle Stereoisomere allein sowie deren Mischungen, insbesondere deren Racemate, erfindungsgemäß.

Die Verbindungen gemäß Formel (I) können neben der Form als freie Säure oder als dessen Salz auch als inneres Salz vorliegen, insbesondere dann, wenn neben der Sulfinat-Gruppe der Formel (I) zusätzlich ein kationischer Substituent (siehe Definition R² und R⁷) im Molekül enthalten ist.

Wenn die Verbindung der Formel (I) als Säure vorliegt, bedeuten die Reste M, M', M" und/oder M^{III} ein Wasserstoffatom. Die Fragmente MO- der Formel (I), M'O- der Formel (III) und M^{II}O- bzw. M^{III}O-gemäß R¹ bzw. R² aus Formel (II) bilden in diesem Fall eine Hydroxygruppe. Wenn die Sulfinsäure der Formel (I) als Salz vorliegt, steht mindestens einer der Reste M, M', M" oder M^{III} für ein Äquivalent eines ein- oder mehrwertigen Kations. Das ein oder mehrwertige Kation M^{z+} mit einer Ladungszahl z von eins oder höher dient lediglich aus Gründen der Elektroneutralität zur Kompensation der einfach negativen Ladung des bei Salzbildung vorliegenden Sulfinat-Fragments 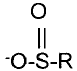 aus Formel (I) bzw. mutatis mutandis aus Formel (III), bzw. der Carboxylat-Fragmente der Reste aus R¹ bzw. R² der Formel (II). Das dafür zu verwendende Äquivalent des entsprechenden Kations beträgt 1/z. Das Fragment MO- der Formel (I) bzw. das Fragment M'O- der Formel (III) stehen im Fall der Salzbildung für die Gruppe: 1/z (M^{z+}) ⁻O- bzw. die Gruppe: 1/z (M'^{z+})⁻O-. Gleiches gilt mutatis mutandis für M" und M^{III}.

Als entsprechende ein- oder mehrwertige Kationen kommen prinzipiell alle Kationen in Frage, die keine Redox-Reaktion mit dem übrigen Sulfinat-Fragment der Formel (I) eingehen. Insbesondere sind dies Metallkationen der physiologisch verträglichen Metalle aus den Gruppen la, Ib, IIa, IIb, IIIb, VIa oder VIII des Periodensystems der Elemente, Ammoniumionen, sowie kationische organische Verbindungen mit quaterniertem Stickstoffatom. Letztere werden beispielsweise durch Protonierung primärer, sekundärer oder tertiärer organischer Amine mit einer Säure, wie z.B. mit Verbindungen der Formel (I) in ihrer sauren Form, oder durch permanente Quaternisierung besagter organischer Amine gebildet. Beispiele dieser kationischen organischen Ammoniumverbindungen sind 2-Ammonioethanol und 2-Trimethylammonioethanol. M, M', M" und M^{III} stehen bevorzugt unabhängig voneinander für ein Wasserstoffatom, ein Ammoniumion, ein Alkalimetallion, für ein halbes Äquivalent eines Erdalkalimetallions oder ein halbes Äquivalent eines Zinkions, besonders bevorzugt für ein Wasserstoffatom, ein Ammoniumion, ein Natriumion, ein Kaliumion, ½ Kalziumion, ½ Magnesiumion oder ½ Zinkion.

Das Äquivalent des gegebenenfalls vorhandenen ein- oder mehrwertigen Anions gemäß Formel (I) wird, analog zur Definition der Kationäquivalente, zur Wahrung der Elektroneutralität durch Formulierung eines stöchiometrischen Koeffizienten kleiner 1 vor der Bezeichnung des Anions beschrieben. Die besagten Anionen werden im Weiteren definitionsgemäß mit An⁻ symbolisiert. Sie werden bevorzugt ausgewählt aus Halogenid, ½ Sulfat, Hydrogensulfat, ½ Carbonat, Hydrogencarbonat, 1/3 Phosphat, ½ Hydrogenphosphat, Dihydrogenphosphat oder p-Toluolsulfonat. Besonders bevorzugt steht das Anion für Chlorid, Bromid, p-Toluolsulfonat oder Hydrogensulfat.

Erfindungsgemäß sind weiterhin solche Sulfinsäurederivate gemäß Formel (I) bevorzugt, in denen die Reste Y und Y' unabhängig voneinander eine Hydroxygruppe oder eine Gruppe
-NH₂ bedeuten.

Sulfinsäurederivate, in denen R¹ für ein Wasserstoffatom oder eine Methylgruppe steht, sind erfindungsgemäß bevorzugt.

Steht der Rest R der Formel (I) für einen Rest der oben genannten Formel (II), dann ergibt sich als bevorzugte erste Ausführungsform aus der Formel (I) das erfindungsgemäße Sulfinsäurederivat der Formel (la), 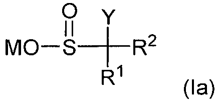 worin M, Y, R¹ und R² die unter den Formeln (I) und (II) definierte Bedeutung haben. Die zuvor erwähnten bevorzugten Definitionen der Reste M, Y und R¹ gelten auch hier, allein oder gemeinsam auf Formel (la) angewendet, als bevorzugt.

Es ist erfindungsgemäß, wenn gemäß einer bevorzugten Ausführungsform der Erfindung der Rest R² gemäß Formel (II) bzw. Formel (la) für einen aliphatischen oder aromatischen Heterozyklus steht, welcher gegebenenfalls substituiert sein kann. Falls die oben genannten ausgewählten aliphatischen oder aromatischen Heterozyklen dieser Ausführungsform substituiert sind, dann sind diese bevorzugt mit mindestens einem Rest aus (C₁ bis C₆)-Alkyl, (C₂ bis C₆)-Alkenyl, (C₁ bis C₆)-Hydroxyalkyl, Aryl-(C₁ bis C₆)-alkyl, Hydroxy, (C₁ bis C₆)-Alkoxy, Amino, Di(C₁ bis C₆)alkylamino, Nitro, Halogen, Carbamoyl, Sulfonamido, Cyano oder Carboxamido, substituiert.

Im Rahmen dieser Ausführungsform ist es wiederum bevorzugt, die aliphatischen oder aromatischen Heterozyklen des Rests R² auszuwählen aus Thienyl, Furyl, Pyrrolyl, Imidazolyl, Thiazolyl, Oxazolyl, Pyrazolyl, Pyridyl, Pyrimidinyl, Pyrazyl, Pyridazyl, Benzimidazolyl, Benzothiazolyl, Benzoxazolyl, Indolyl, Chinolinyl, Chinoxalinyl oder Chinazolinyl, welche gegebenenfalls substituiert sein können, bevorzugt mit den oben genannten Substituenten, besonders bevorzugt mit mindestens einem Rest aus (C₁ bis C₆)-Alkyl, (C₁ bis C₆)-Hydroxyalkyl, Hydroxy, Amino, (C₁ bis C₆)-Alkoxy, Carboxy, Halogenatom, Nitrogruppe oder Sulfonsäurerest.

Dabei sind besonders bevorzugte Sulfinsäurederivate dieser Ausführungsform Verbindungen der nachfolgenden Formeln (Ia-1) bis (la-4) 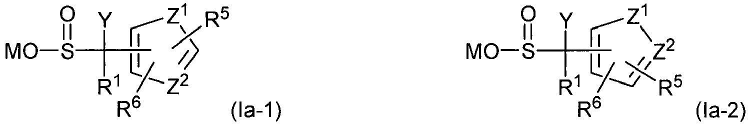 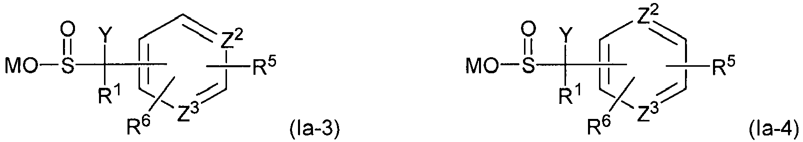 worin
- M, Y und R¹: die unter Formel (I) definierten Bedeutungen haben,
- Z¹: für ein Sauerstoffatom, ein Schwefelatom oder eine Gruppe N-R^{IV} steht, worin R^{IV} ein Wasserstoffatom, eine (C₁ bis C₆)-Alkylgruppe, eine (C₂ bis C₆)-Alkenylgruppe, eine (C₁ bis C₆)-Hydroxyalkylgruppe oder eine Aryl-(C₁ bis C₆)-alkylgruppe bedeutet,
- Z² und Z³: unabhängig voneinander für eine Gruppe CH oder für ein Stickstoffatom stehen,
- R⁵ und R⁶: unabhängig voneinander stehen für ein Wasserstoffatom, eine (C₁ bis C₆)-Alkylgruppe, eine (C₂ bis C₆)-Alkenylgruppe, eine (C₁ bis C₆)-Hydroxyalkylgruppe, eine Hydroxygruppe, eine (C₁ bis C₆)-Alkoxygruppe, eine Aminogruppe, eine Di(C₁ bis C₆)alkylaminogruppe, eine Nitrogruppe, eine Halogenatom, eine Carbamoylgruppe, eine Sulfonamidogruppe, eine Cyanogruppe oder eine Carboxamidogruppe oder gemeinsam eine Benzanellierung bilden, welche wiederum substituiert sein kann.

Die Gruppe N-R^{IV} aus Rest Z¹ bildet im Ring des Heterozyklus eine Azandiylgruppe -NR^{IV}-. Die Gruppe CH der Reste Z² bzw. Z³ bildet im Ring des Heterozyklus eine Methanylylidengruppe =CH-, die selbstverständlich auch mit einem der Reste R⁵ bzw. R⁶ substituiert sein kann.

Wenn in den Formeln (Ia-1) bzw. (la-2) Z¹ für ein Sauerstoffatom, Z² für eine Gruppe CH und Y für eine Hydroxygruppe steht, so ist es erfindungsgemäß bevorzugt, wenn in den Formeln (Ia-1) bzw. (la-2) R⁵ und R⁶ nicht gleichzeitig für ein Wasserstoffatom stehen.
Die zuvor erwähnten bevorzugten Definitionen der Reste M, Y und R¹ gelten auch hier, allein oder gemeinsam, auf Formeln (la-1) bis (la-4) angewendet, als bevorzugt.

Die Benzanellierung aus den Resten R⁵ und R⁶ der Formel (Ia-1) bis (la-4) ist, wenn sie substituiert ist, bevorzugt mit mindestens einem Rest aus (C₁ bis C₆)-Alkyl, (C₂ bis C₆)-Alkenyl, (C₁ bis C₆)-Hydroxyalkyl, Aryl-(C₁ bis C₆)-alkyl, Hydroxy, (C₁ bis C₆)-Alkoxy, Amino, Di(C₁ bis C₆)alkylamino, Nitro, Halogen, Carbamoyl, Sulfonamido, Cyano oder Carboxamido substituiert.

R⁵ und R⁶ gemäß Formeln (Ia-1) bis (la-4) stehen besonders bevorzugt für ein Wasserstoffatom, eine Hydroxygruppe, eine (C₁ bis C₆)-Alkylgruppe, eine (C₁ bis C₆)-Alkoxygruppe, eine (C₁ bis C₆)-Hydroxyalkylgruppe, Halogenatom oder Nitrogruppe.

Wenn die Reste R¹ und R² gemäß Formel (II) im Rahmen der ersten Ausführungsform gemeinsam mit dem Restmolekül einen aliphatischen 5-, 6-, oder 7-gliederigen Ring bilden, welcher mindestens ein kationisches, quaterniertes Stickstoffatom als Heteroatom enthält wobei die kationische Ladung gegebenenfalls durch die ein Äquivalent eines ein oder mehrwertigen Anions kompensiert wird, sind folgende Verbindungen gemäß Formeln (la-5) und (la-6) bevorzugt, 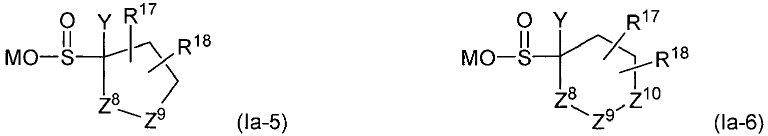 worin
- Y: die unter Formel (I) beschriebenen Definitionen bedeutet,
- M: die unter Formel (I) beschriebenen Definitionen oder eine negative Ladung bedeutet,
- Z⁸, Z⁹ und Z¹⁰: einer dieser Reste eine Azoniumdiyl-Gruppe N⁺R^{V}R^{VI} bedeutet
mit R^{V} und R^{VI} stehen unabhängig voneinander für eine (C₁ bis C₆)-Alkylgruppe, eine (C₂ bis C₆)-Alkenylgruppe, eine (C₁ bis C₆)-Hydroxyalkylgruppe oder eine Aryl-(C₁ bis C₆)-alkylgruppe,
und die übrigen dieser Reste für eine CH₂-Gruppe stehen,
- R¹⁷ und R¹⁸: unabhängig voneinander ein Wasserstoffatom, eine (C₁ bis C₆)-Alkylgruppe, eine Hydroxygruppe, ein Halogenatom oder eine Carboxygruppe bedeuten,
mit der Maßgabe, dass ein Äquivalent eines ein- oder mehrwertigen Anions zugegen ist, wenn M keine negative Ladung bedeutet.

Die CH₂-Gruppe bildet im Ring des Heterozyklus ein Methandiylfragment -CH₂-, das selbstverständlich auch mit einem der Reste R¹⁷ bzw. R¹⁸ substituiert sein kann.

Die zuvor erwähnten bevorzugten Definitionen der Reste M und Y gelten auch hier, allein oder gemeinsam, auf Formeln (la-5) und (la-6) angewendet, als bevorzugt. Die unter der Maßgabe beschriebene Bedingung beschreibt die Ausbildung eines inneren Salzes, das ebenso eine bevorzugte Ausführungsform darstellt.

Wenn der Rest R² der Formel (II) für einen Rest der oben genannten Formel (IV) steht, dann ergibt sich aus der Formel (I) das erfindungsgemäße Sulfinsäurederivat gemäß Formel (Ia-7), 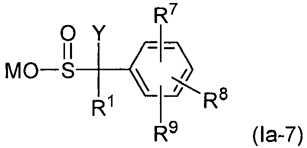 worin,
M, Y, R¹, R⁷, R⁸ und R⁹ die unter Formel (I) genannten Bedeutungen haben.

Es sind solche Verbindungen der Formel (la-7) erfindungsgemäß bevorzugt, bei welchen der Rest R⁷ eine Carboxygruppe, eine Sulfonsäuregruppe oder eine Gruppe -N⁺R^{I}R^{II}R^{III},
mit R^{I}, R" und R^{III} stehen unabhängig voneinander für eine (C₁ bis C₆)-Alkylgruppe, eine (C₂ bis C₆)-Alkenylgruppe, eine Aryl(C₁ bis C₆)-alkylgruppe oder eine (C₁ bis C₆)-Hydroxyalkylgruppe, bedeutet und die Reste R⁸ und R⁹ für ein Wasserstoffatom stehen.

Weiterhin erfindungsgemäß bevorzugt eingesetzte Verbindungen der Formel (I) sind Verbindungen der Formel (la-8) 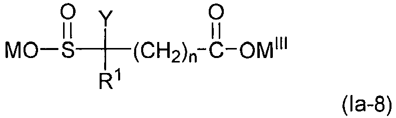 worin M, Y, R¹, n und M^{III} die unter Formel (I) und (II) genannten Bedeutungen haben.

Es sind solche Verbindungen der Formel (la-8) erfindungsgemäß bevorzugt, bei welchen Y eine Hydroxy- oder Aminogruppe bedeutet.

Bevorzugt steht n in Formel (la-8) für eine Zahl 0, 1 oder 2.

Steht der Rest R der Formel (I) für einen Rest der oben genannten Formel (III), dann ergibt sich aus der Formel (I) als bevorzugte zweite Ausführungsform der Erfindung das erfindungsgemäße Sulfinsäurederivat der Formel (Ib), 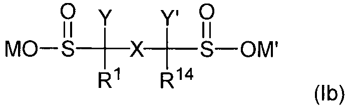 worin M, M', Y, Y', X, R¹ und R¹⁴ die unter den Formeln (I) und (III) definierte Bedeutung haben. Die zuvor erwähnten bevorzugten Definitionen der Reste M, M', Y, Y', X, R¹ und R¹⁴ gelten auch hier, allein oder gemeinsam auf Formel (Ib) angewendet, als bevorzugt.

Der Rest X steht bevorzugt für einen organischen Rest mit zwei freien Valenzen. Als erfindungsgemäße Reste eignen sich prinzipiell alle Organodiyl-Reste, wie z.B. aliphatische oder alizyklische, aromatische oder heteroaromatische Diyl-Reste. Der besagte organische Rest mit zwei freien Valenzen X gemäß Formel (III) bzw. Formel (Ib) wird bevorzugt aus der Gruppe ausgewählt, die gebildet wird aus gegebenenfalls substituierten Resten aus Arendiyl, Heteroarendiyl, Alkandiyl, Alkendiyl, Cycloalkandiyl, Cycloalkendiyl und Di((C₁ bis C₆)alkylen)-substituierten carbozyklischen oder heterozyklischen Gruppen, die aliphatisch oder aromatisch sind. Falls die oben genannten ausgewählten Reste dieser Ausführungsform substituiert sind, dann sind diese bevorzugt mit mindestens einem Rest aus (C₁ bis C₆)-Alkyl, (C₂ bis C₆)-Alkenyl, (C₁ bis C₆)-Hydroxyalkyl, Aryl-(C₁ bis C₆)-alkyl, Hydroxy, (C₁ bis C₆)-Alkoxy, Amino, Di(C₁ bis C₆)alkylamino, Nitro, Halogen, Carbamoyl, Sulfonamido, Cyano oder Carboxamido substituiert.

Es ist erfindungsgemäß besonders bevorzugt, wenn der Rest X der Formel (III) bzw. der Formel (Ib) für einen organischen Rest mit zwei freien Valenzen steht, der aus einer (C₁ bis C₆)-Alkandiylgruppe oder aus Resten der Formeln (VII) bis (XI) ausgewählt wird, 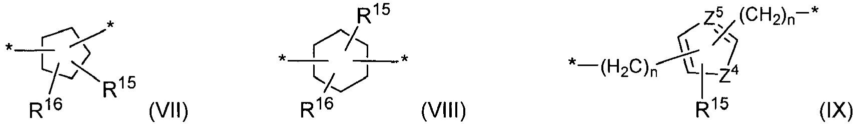 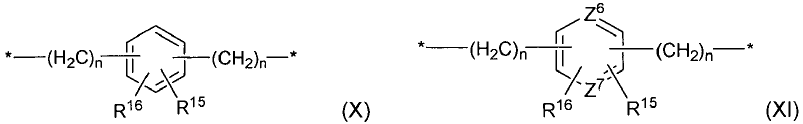 worin bedeuten
- R¹⁵ und R¹⁶: unabhängig voneinander für ein Wasserstoffatom, ein Halogenatom, eine (C₁ bis C₆)-Alkylgruppe oder eine Carboxygruppe,
- n: eine ganze Zahl von 0 bis 6,
- Z⁴: eine Gruppe CH₂, ein Sauerstoffatom, ein Schwefelatom oder eine Gruppe NR', mit R' = Wasserstoffatom, eine (C₁ bis C₆)-Alkylgruppe, eine (C₁ bis C₆)-Hydroxyalkylgruppe oder eine (C₂ bis C₆)-Polyhydroxyalkylgruppe,
- Z⁵, Z⁶ und Z⁷: unabhängig voneinander eine Gruppe CH oder ein Stickstoffatom.

Die jeweiligen Gruppen CH bzw. CH₂ der Reste Z⁴, Z⁵, Z⁶ oder Z⁷ können selbstverständlich erfindungsgemäß ebenso mit allen erfindungsgemäß im Rahmen der Definition der betroffenen Formel aus den Formeln (VII) bis (XI) möglichen Substituenten substituiert sein.

Falls der organische Rest mit zwei freien Valenzen X aus den Formeln (VII) und (VIII) ausgewählt wird, sind die Reste 2-Chlor-cyclopentan-1,3-diyl, 2-Brom-cyclopentan-1,3-diyl, 2-Chlor-cyclohexan-1,3-diyl und 2-Brom-cyclohexan-1,3-diyl bevorzugte Vertreter.

Im Folgenden sollen Beispiele für die als Substituenten im Rahmen dieser Anmeldung genannten Gruppen bzw. Reste erwähnt werden. Beispiele für (C₁ bis C₆)-Alkylreste sind lineare, verzweigte oder zyklische (C₁ bis C₆)-Alkylgruppen, wobei lieneare oder verzweigte (C₁ bis C₆)-Alkylgruppen bevorzugt sind. Insbesondere sind die Gruppen Methyl, Ethyl, Propyl, Isopropyl, n-Butyl, sec-Butyl, tert.-Butyl, n-Pentyl und n-Hexyl geeignet. Beispiele für entsprechend geeignete zyklische Alkylgruppen sind Cyclopentyl und Cyclohexyl.
Beispiele für bevorzugte (C₂ bis C₆)-Alkenylreste sind Vinyl, Allyl und Butenyl.
Erfindungsgemäß bevorzugte (C₁ bis C₆)-Alkoxyreste sind beispielsweise eine Methoxy- oder eine Ethoxygruppe.
Die Methoxycarbonyl-, Ethoxycarbonyl-, n-Propoxycarbonyl-, Isopropoxycarbonyl-, n-Butoxycarbonyl-, sec-Butoxycarbonyl- und tert-Butoxycarbonylgruppe sind Beispiele für (C₁ bis C₆)-Alkoxycarbonylgruppen; die Methoxycarbonyl- und die Ethoxycarbonylgruppe sind dabei besonders bevorzugt.
Die 2-Methoxycarbonylethyl-, 2-Ethoxycarbonylethyl-, 2-Propoxycarbonylethyl-, 2-Isopropoxycarbonylethyl-, 2-Butoxycarbonylethyl-, 2-sec-Butoxycarbonylethyl-, 2-tert-Butoxycarbonylethyl-, 3-Methoxycarbonylpropyl-, 3-Ethoxycarbonylpropyl-, 3-Propoxycarbonylpropyl-, 3-Isopropoxycarbonylpropyl-, 3-Butoxycarbonylpropyl-, 3-sec-Butoxycarbonylpropyl- und 3-tert-Butoxycarbonylpropylgruppe sind Beispiele für (C₁ bis C₆)-Alkoxycarbonyl-(C₁ bis C₆)-alkylgruppen. Beispiele für erfindungsgemäße Cyano-(C₁ bis C₆)-alkylgruppen sind Cyanomethyl, 2-Cyanoethyl, 3-Cyanopropyl, 4-Cyanobutyl und 5-Cyanopentyl.
Weiterhin können als bevorzugte Beispiele für eine (C₁ bis C₆)-Monohydroxyalkylgruppe eine Hydroxymethyl-, eine 2-Hydroxyethyl-, eine 2-Hydroxypropyl, eine 3-Hydroxypropyl-, eine 4-Hydroxybutylgruppe, eine 5-Hydroxypentyl- und eine 6-Hydroxyhexylgruppe genannt werden. Eine 2-Hydroxyethylgruppe ist besonders bevorzugt.
Beispiele für eine (C₂ bis C₆)-Polyhydroxyalkylgruppe sind die 2,3-Dihydroxypropylgruppe, 3,4-Dihydroxybutylgruppe und die 2,4-Dihydroxybutylgruppe.
Die Methoxyethyl-, Ethoxyethyl-, Methoxypropyl-, Methoxybutyl-, Ethoxybutyl- und die Methoxyhexylgruppe sind Beispiele für erfindungsgemäße (C₁ bis C₆)-Alkoxy-(C₁ bis C₆)-alkylgruppen. Eine bevorzugte Hydroxy-(C₁ bis C₆)-alkoxygruppe ist die 2-Hydroxyethoxygruppe. Bevorzugte Arylgruppen sind Phenyl, Naphthyl und Biphenyl.
Bevorzugte Heteroarylgruppen sind Thienyl, Furyl, Pyrrolyl, Imidazolyl, Thiazolyl, Oxazolyl, Pyrazolyl, Pyridyl, Pyrimidinyl, Pyrazyl, Pyridazyl, Benzimidazolyl, Benzothiazolyl, Benzoxazolyl, Indolyl, Chinolinyl, Chinoxalinyl und Chinazolinyl.
Die Trifluomethyl- und die Pentafluorethylgruppe sind bevorzugte Perfluor-(C₁ bis C₆)-alkylgruppen. Beispiele für Halogenatome sind F-, Cl-, Br- oder I-Atome, wobei Cl- und Br-Atome ganz besonders bevorzugt sind.
Bevorzugte Aryl-(C₁ bis C₆)-alkylgruppen sind Benzyl und 2-Phenylethyl.
Die Trimethylammonium- und Diethylmethylammonium- sind Beispiele für eine Gruppe -N⁺R^{I}R^{II}R^{III}. Die 2-Trimethylammoniumethyl- ist ein Beispiel für eine N,N,N-Tri[(C₁ bis C₆)-akyl]ammonium-(C₁ bis C₆)-alkylgruppe.
Eine bevorzugte (C₁ bis C₆)-Carboxyalkylgruppe ist die 3-Carboxypropylgruppe.
Die weiteren verwendeten Begriffe leiten sich erfindungsgemäß von den hier gegebenen Definitionen ab.

Ganz besonders bevorzugte Vertreter der Sulfinsäurederivate gemäß Formel (I) sind die Sulfinsäuren gemäß folgender Liste oder deren Salze mit einem Äquivalent mindestens eines ein- oder mehrwertigen Kations:

| Name: | korrespondierende Struktur: |
|---|---|
| 2-Furyl(amino)methansulfinsäure, | 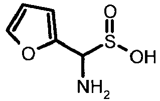 |
| Amino(thien-2-yl)methansulfinsäure, | 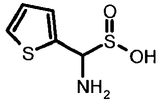 |
| Amino(1*H*-imidazol-2-yl)methansulfinsäure, | 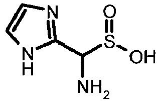 |
| Amino(1,3-thiazol-2-yl)methansulfinsäure, | 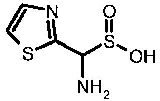 |
| Amino(1,3-oxazol-2-yl)methansulfinsäure, | 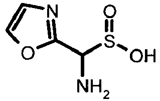 |
| Amino(1*H*-pyrrol-2-yl)methansulfinsäure, | 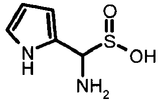 |
| Amino(hydroxyl-2-yl)methansulfinsäure, | 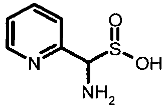 |
| Amino(hydroxyl-4-yl)methansulfinsäure, | 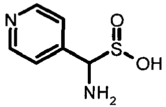 |
| Amino[3-hydroxy-5-(hydroxymethyl)-2-methylpyridin-4-yl]methansulfinsäure, | 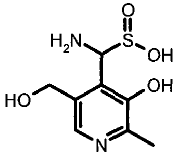 |
| Amino(chinolin-2-yl)methansulfinsäure, | 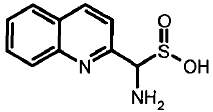 |
| Amino(chinolin-4-yl)methansulfinsäure, | 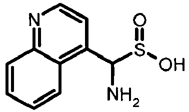 |
| 1*H*-Benzimidazol-2-yl(amino)methansulfinsäure, | 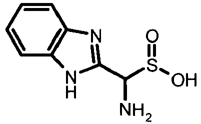 |
| 1,3-Benzothiazol-2-yl(amino)methansulfinsäure, | 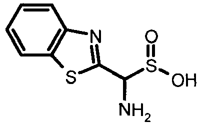 |
| 1,3-Benzoxazol-2-yl(amino)methansulfinsäure, | 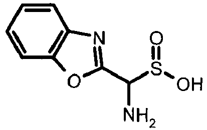 |
| Hydroxy(thien-2-yl)methansulfinsäure, | 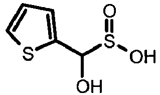 |
| Hydroxy(thien-3-yl)methansulfinsäure | 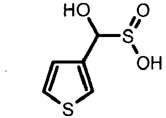 |
| (3-Bromthien-2-yl)(hydroxyl)methansulfinsäure, | 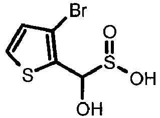 |
| Hydroxy(1H-imidazol-2-yl)methansulfinsäure, | 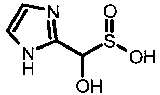 |
| Hydroxy(1H-imidazol-5-yl)methansulfinsäure, | 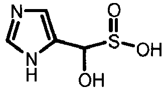 |
| Hydroxy(1-methyl-5-nitro-1*H*-imidazol-2-yl)methansulfinsäure, | 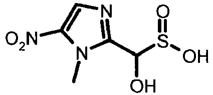 |
| Hydroxy(1,3-thiazol-2-yl)methansulfinsäure, | 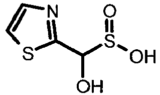 |
| Hydroxy(1,3-oxazol-2-yl)methansulfinsäure, | 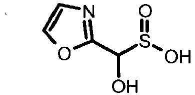 |
| Hydroxy(1*H*-pyrrol-2-yl)methansulfinsäure, | 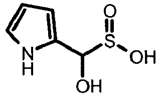 |
| Hydroxy(hydroxyl-2-yl)methansulfinsäure, | 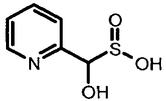 |
| Hydroxy(hydroxyl-4-yl)methansulfinsäure, | 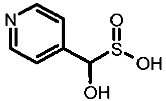 |
| Hydroxy[3-hydroxy-5-(hydroxymethyl)-2-methylpyridin-4-yl]-methansulfinsäure, | 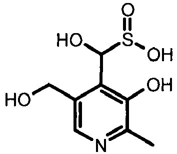 |
| Hydroxy(chinolin-2-yl)methansulfinsäure, | 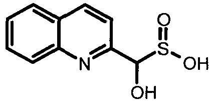 |
| Hydroxy(chinolin-4-yl)methansulfinsäure, | 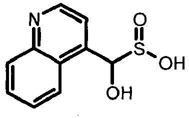 |
| 1*H*-Benzimidazol-2-yl(hydroxyl)methansulfinsäure, | 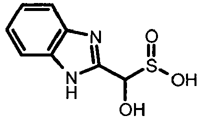 |
| 1,3-Benzothiazol-2-yl(hydroxyl)methansulfinsäure, | 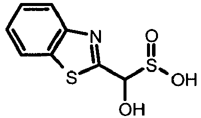 |
| 1,3-Benzoxazol-2-yl(hydroxy)methansulfinsäure, | 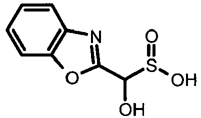 |
| 1,2-Dihydroxyethan-1,2-disulfinsäure, | 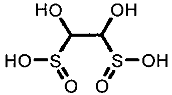 |
| 1,3-Dihydroxypropan-1,3-disulfinsäure, | 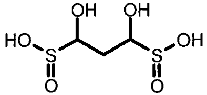 |
| Hydroxy{4-[hydroxy(sulfino)methyl]phenyl}methansulfinsäure, | 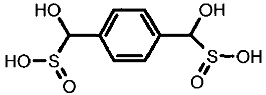 |
| {2-Chlor-3-[hydroxy(sulfino)methyl]cyclohexyl}-(hydroxy)methansulfinsäure, | 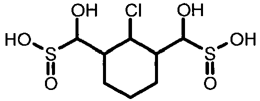 |
| {2-Chlor-3-[hydroxyl(sulfino)methyl]cyclopentyl}-(hydroxyl)methansulfinsäure, | 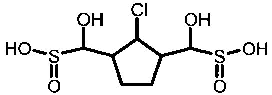 |
| Hydroxy{5-[hydroxy(sulfino)methyl]thien-2-yl}methansulfinsäure, | 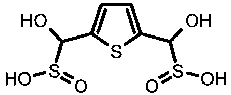 |
| Hydroxy{2-[hydroxy(sulfino)methyl]phenyl}methansulfinsäure, | 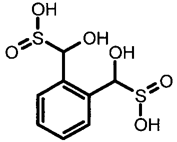 |
| Hydroxy{3-[hydroxy(sulfino)methyl]phenyl}methansulfinsäure, | 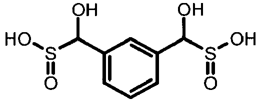 |
| 1,5-Dihydroxypentan-1,5-disulfinsäure, | 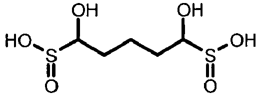 |
| 4-Hydroxy-4-sulfinobutansäure, | 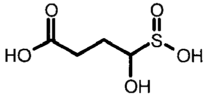 |
| 1-Hydroxy-4-methoxy-4-oxobutan-1-sulfinsäure, | 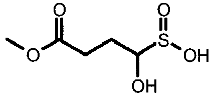 |
| 1-Hydroxy-4-ethoxy-4-oxobutan-1-sulfinsäure, | 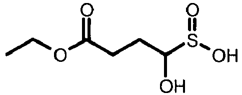 |
| 4-Hydroxy-4-sulfinopentansäure, | 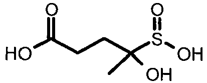 |
| 2-Hydroxy-5-methoxy-5-oxopentan-2-sulfinsäure, | 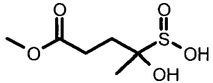 |
| 2-Hydroxy-5-ethoxy-5-oxopentan-2-sulfinsäure, | 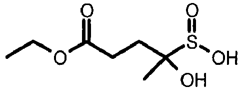 |
| 4-Hydroxy-4-sulfinobut-2-ensäure, | 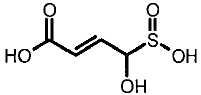 |
| 4-Hydroxy-4-sulfinopent-2-ensäure, | 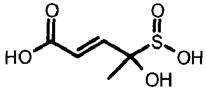 |
| 5-Hydroxy-5-sulfinopentansäure, | 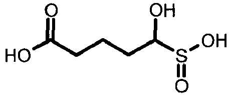 |
| 1-Hydroxy-5-methoxy-5-oxopentan-1-sulfinsäure, | 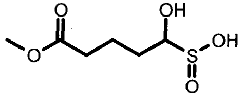 |
| 1-Hydroxy-5-ethoxy-5-oxopentan-1-sulfinsäure, | 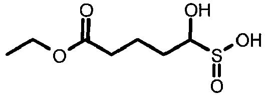 |
| 5-Hydroxy-5-sulfinohexansäure, | 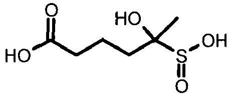 |
| 2-Hydroxy-6-methoxy-6-oxohexan-2-sulfinsäure, | 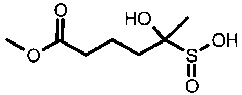 |
| 2-Hydroxy-6-ethoxy-6-oxohexan-2-sulfinsäure, | 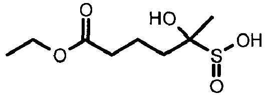 |
| 4-[Hydroxy(sulfino)methyl]benzoesäure, | 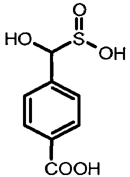 |
| 4-[Amino(sulfino)methyl]benzoesäure, | 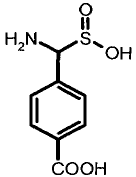 |
| {4-[Hydroxy(sulfino)methyl]phenoxy}-essigsäure, | 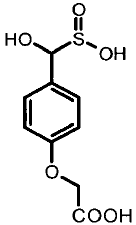 |
| {4-[Amino(sulfino)methyl]phenoxy}-essigsäure, | 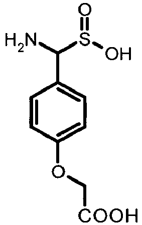 |
| 3-Hydroxy-4-[hydroxy(sulfino)methyl]benzoesäure, | 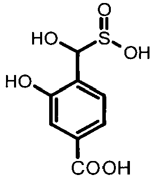 |
| 3-Hydroxy-4-[amino(sulfino)methyl]benzoesäure, | 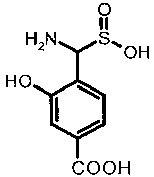 |
| (4-Cyanophenyl)(hydroxy)-methansulfinsäure, | 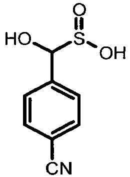 |
| (4-Cyanophenyl)(amino)-methansulfinsäure, | 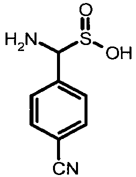 |
| (4-Nitrophenyl)(hydroxy)-methansulfinsäure, | 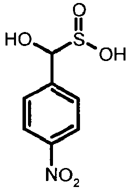 |
| (4-Nitrophenyl)(amino)-methansulfinsäure, | 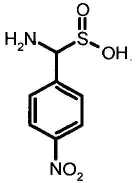 |
| Salz des 4-Hydroxy-1,1-dimethyl-4-sulfinopiperidiniums mit Anals Äquivalent eines ein- oder mehrwertigen Anions, | 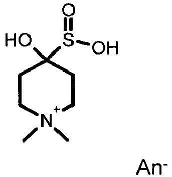 |
| Salz des 1-Allyl-4-hydroxy-1-methyl-4-sulfinopiperidiniums mit An⁻ als Äquivalent eines ein- oder mehrwertigen Anions, | 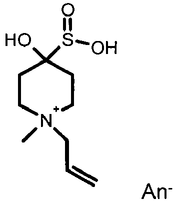 |
| 4-Hydroxy-1,1-dimethylpiperidinium-4-sulfinat, | 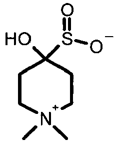 |
| 1-Allyl-4-hydroxy-1-methylpiperidinium-4-sulfinat, | 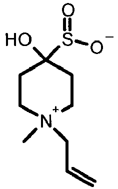 |
| 1-Hydroxy-2-(trimethylammonio)ethansulfinat, | 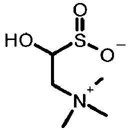 |
| Hydroxy[4-(trimethylammonio)phenyl]methansulfinat, | 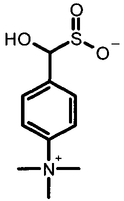 |
| 4-[Hydroxy(sulfino)methyl]benzolsulfonsäure und | 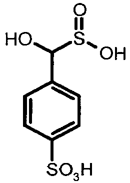 |
| 2-[Hydroxy(sulfino)methyl]benzolsulfonsäure | 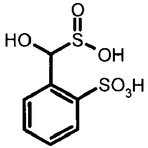 |
| 2-Hydroxy-2-sulfino-essigsäure | 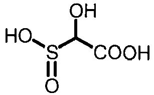 |
| 2-Amino-2-sulfino-essigsäure | 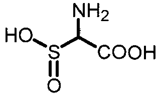 |
| 2-Hydroxy-2-sulfino-propionsäure | 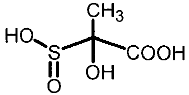 |
| 2-Amino-2-sulfino-propionsäure | 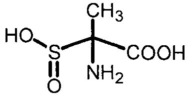 |
| 3-Hydroxy-3-sulfinato-propionsäure | 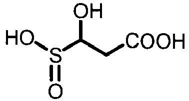 |
| 3-Amino-3-sulfinato-propionsäure | 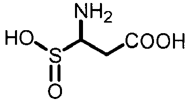 |
| 2-Hydroxy-2-sulfinato-essigsäureethylester | 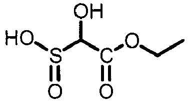 |
| 2-Amino-2-sulfinato-essigsäureethylester | 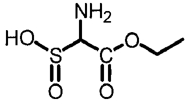 |
| 1-Hydroxy-2-methoxy-2-oxoethansulfinsäure | 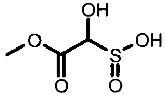 |
| 2-Amino-1-hydroxy-2-oxoethansulfinsäure | 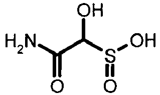 |
| 2-(Dimethylamino)-1-hydroxy-2-oxoethansulfinsäure | 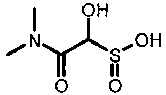 |
| Hydroxy(sulfino)methansulfonsäure | 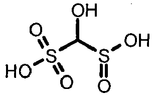 |

Für die bevorzugten ein- oder mehrwertigen Kationen aller Salze der zuvor genannten Verbindungen gilt das zuvor Gesagte.

Bevorzugt enthält das erfindungsgemäße Mittel mindestens ein Sulfinsäurederivat der Formel (I) in einer Menge von 0,01 bis 20 Gew.-%, besonders bevorzugt von 1 bis 20 Gew.-%, jeweils bezogen auf das Gewicht des Mittels.

Nichtionische Tenside sind grenzflächenaktive Stoffe mit einer ungeladenen, bei neutralem pH-Wert keine Ionenladung tragenden, polaren, hydrophilen Kopfgruppe, die an Grenzflächen adsorbieren und oberhalb der kritischen Micellbildungskonzentration zu neutralen Micellen aggregieren.

Die erfindungsgemäßen Mittel enthalten als nichtionisches Tensid mindestens einen Vertreter aus der Gruppe, die gebildet wird, aus Anlagerungsprodukten von 2 bis 30 Mol Ethylenoxid und/oder 0 bis 5 Mol Propylenoxid an lineare Fettalkohole mit 8 bis 22 C-Atomen.

Bei den Tensiden, die Anlagerungsprodukte von Ethylen- und/oder Propylenoxid an Fettalkohole darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden.
Die Anlagerungsprodukte, die nur wenig freien Fettalkohol enthalten und eine eingeengte Homologenverteilung aufweisen (sogenannte "narrow range ethoxylates"), wie sie z.B. nach dem in DE 38 43 713 A1 beschriebenen Verfahren zugänglich sind, können Vorteile in den erfindungsgemäßen Mitteln aufweisen.

Die erfindungsgemäßen Mittel enthalten die nichtionischen Tenside bevorzugt in Mengen von 0,1 bis 25 Gew.-%, insbesondere 0,5 bis 15 Gew.-%, jeweils bezogen auf das gesamte Mittel.

Es ist weiterhin bevorzugt, wenn in dem erfindungsgemäßen Mittel das Gewichtsverhältnis der Sulfinsäurederivate der Formel (I) zu den nichtionischen Tensiden von 1 zu 2000 bis 10 zu 1, besonders bevorzugt von 1 zu 200 bis 2,5 zu 1, ganz besonders bevorzugt von 1 zu 50 bis 1 zu 1, beträgt.

Zusätzlich können die erfindungsgemäßen Mittel mindestens eine Verbindung, ausgewählt aus den Aldehyden oder den Ketonen, enthalten. Bevorzugte Verbindungen aus den Aldehyden oder den Ketonen werden ausgewählt aus mindestens einem Vertreter aus der Gruppe
- Oxocarbonsäuren oder deren Salze,
- Oxocarbonsäureester,
- cyclische, lineare oder verzweigte aliphatische Aldehyde,
- cyclische, lineare oder verzweigte aliphatische Ketone,
- mono- bis polyhydroxyfunktionalisierte Aldehyde,
- mono- bis polyhydroxyfunktionalisierte Ketone,
- alicyclische, aromatische oder heterocyclische Aldehyde sowie
- alicyclische, aromatische oder heterocyclische Ketone.

Oxocarbonsäuren sind organische Verbindungen, die neben mindestens einer Carboxylgruppe eine Carbonylgruppe tragen und sind somit Aldehyd- bzw. Ketocarbonsäuren. Besonders bevorzugt werden die Oxocarbonsäuren ausgewählt aus mindestens einem Vertreter aus der Gruppe Glyoxalsäure, Acetessigsäure, 3-Oxoglutarsäure, 4-Oxovaleriansäure und Brenztraubensäure bzw. den Salzen der vorgenannten Säuren. Die Oxocarbonsäureester werden bevorzugt ausgewählt aus (C₁ bis C₆)-Alkylestern der erfindungsgemäß bevorzugten Oxocarbonsäuren.

Unter cyclischen, linearen oder verzweigten aliphatischen Aldehyden sind erfindungsgemäß aliphatische Aldehyde zu verstehen, deren Formylgruppe(n) nicht in Konjugation mit einem aromatischen π-Elektronen-System stehen. Die entsprechenden Aldehyde dürfen aromatische Reste tragen, solange die π-Elektronen der Formylgruppe(n) nicht über ein solches aromatisches System delokalisiert sein können. Bevorzugte cyclische, lineare oder verzweigte aliphatische Aldehyde sind gesättigt oder ungesättigt und werden besonders bevorzugt ausgewählt aus mindestens einem Vertreter aus der Gruppe Formaldehyd, Acetaldehyd, Glyoxal, Propionaldehyd, Butanal, Pentanal, Isopentanal, Hexanal, Cyclohexanal, Heptanal, Octanal, Malondialdehyd, Glutaraldehyd, 2-Methylpentanal, 2-Ethylhexanal, 3,5,5-Trimethylhexanal, 2-Ethylbutyraldehyd, 2-Methylbutyraldehyd, Isobutyraldehyd, 3-Phenylpropanal, 3-(4-Methylphenyl)propanal, 3-(4-Methoxyphenyl)propanal, 3-(2-Methoxyphenyl)propanal, 2-Butenal, Acrolein, 3-Methyl-2-butenal, 3,7-Dimethyl-2,6-octadienal, 2,4-Pentadienal, 3,7-Dimethyl-6-octenal, 2,4-Dimethyl-3-cyclohexencarboxaldehyd und 2,6-Nonadienal.

Unter cyclischen, linearen oder verzweigten aliphatischen Ketonen sind erfindungsgemäß aliphatische Ketone zu verstehen, deren Ketogruppe(n) nicht in Konjugation mit einem aromatischen π-Elektronen-System stehen. Die entsprechenden Ketone dürfen jedoch aromatische Reste tragen, solange die π-Elektronen der Ketogruppe(n) nicht über ein solches aromatisches System delokalisiert sein können. Bevorzugte cyclische, lineare oder verzweigte aliphatische Ketone sind gesättigt oder ungesättigt und ausgewählt aus mindestens einem Vertreter aus der Gruppe Aceton, 2-Butanon, 2-Pentanon, 3-Pentanon, 2-Hexanon, 3-Hexanon, Butan-2,4-dion, Pentan-2,4-dion, Hexan-2,5-dion, Cyclohexanon, Cyclopentanon, 4-Methylpentan-2-on, 5-Methyl-3-hexen-2-on, 2-(3-Oxopropyl)benzoesäuremethylester und 4-(3-Oxopropyl)benzoesäuremethylester.

Bevorzugt verwendbare monohydroxyfunktionalisierte Aldehyde werden ausgewählt aus mindestens einem Vertreter aus der Gruppe 1-Hydroxypropanal, 5-Hydroxypentanal, 3,7-Dimethyl-7-hydroxyoctanal, Hydroxyisohexyl-3-cyclohexen-1-carboxaldehyd und 2,6,6-Trimethyl-1,3-cyclohexadien-1-carboxaldehyd.

Polyhydroxyfunktionalisierte Aldehyde sind erfindungsgemäß bevorzugt die sogenannten Aldosen und werden besonders bevorzugt ausgewählt aus mindestens einem Vertreter aus der Gruppe 2,3-Dihydroxypropionaldehyd, D-Erythrose, D-Threose, D-Ribose, D-Arabinose, D-Lyxose, D-Xylose, D-Allose, D-Altrose, D-Galactose, D-Glucose, D-Idose, D-Mannose, D-Rhamnose und D-Talose, sowie den L-Konfigurationen L-Erythrose, L-Threose, L-Ribose, L-Arabinose, L-Lyxose, L-Xylose, L-Allose, L-Altrose, L-Galactose, L-Glucose, L-Idose, L-Mannose, L-Rhamnose und L-Talose.

Monohydroxyfunktionalisierte Ketone, die sich erfindungsgemäß besonders eignen, werden bevorzugt ausgewählt aus mindestens einem Vertreter aus der Gruppe 1-Hydroxy-2-propanon, 1-Hydroxy-2-butanon, 3-Hydroxy-2-butanon und Benzoin.

Polyhydroxyfunktionalisierte Ketone sind erfindungsgemäß bevorzugt die sogenannten Ketosen und werden besonders bevorzugt ausgewählt aus mindestens einem Vertreter aus der Gruppe 1,3-Dihydroxyaceton, D-Psicose, D-Fructose, D-Sorbose, D-Tagatose, D-Ribulose, D-Xylulose, D-Erythrulose sowie den L-Konfigurationen L-Psicose, L-Fructose, L-Sorbose, L-Tagatose, L-Ribulose, L-Xylulose, L-Erythrulose.

Bevorzugte alicyclische, aromatische oder heterocyclische Ketone werden bevorzugt ausgewählt aus mindestens einem Vertreter aus der Gruppe
- Benzylidenketone, insbesondere den Benzylidenketonen aus der Patentanmeldung DE-A1-19717281 (siehe DE-A1-19717281: Formel (I) des 1. Anspruchs und die Vertreter gemäß 4. Anspruch), auf die ausdrücklich Bezug genommen wird,
- Imidazolin-2,4-dion und dessen Derivaten, insbesondere Allantoin und/oder 5,5-Dimethylhydantoin.

Bevorzugte alicyclische, aromatische oder heterocyclische Aldehyde werden bevorzugt ausgewählt aus mindestens einem Vertreter aus der Gruppe
- Benzaldehyd und seinen Derivaten,
- Zimtaldehyd und seinen Derivaten sowie
- Naphthaldehyd und seinen Derivaten.

Alle Aldehyd bzw. Ketoverbindungen sollen aus physiologisch verträglichen bzw. nicht toxischen Verbindungen ausgewählt werden, wenn das erfindungsgemäße Mittel als kosmetisches Mittel Verwendung finden soll.

Die Verbindungen, ausgewählt aus Aldehyden oder Ketonen, sind erfindungsgemäß bevorzugt in einer Menge von 0,1 Gew.-% bis 20 Gew.-%, insbesondere von 0,5 Gew.-% bis 10 Gew.-%, jeweils bezogen auf das Gewicht des anwendungsbereiten Entfärbemittels, in dem Entfärbemittel enthalten.

Es ist ebenso erfindungsgemäß bevorzugt, wenn das erfindungsgemäße Mittel zusätzlich mindestens ein Redukton enthält. Unter einem Redukton versteht der Fachmann reduktiv wirkende Endiol-Verbindungen, die durch Substitution in α-Stellung stabilisiert sind und die der Tautomerie unterliegen. Die wichtigsten erfindungsgemäß einsetzbaren Reduktone sind Ascorbinsäure, Isoascorbinsäure, 2,3-Dihydroxy-2-propendial und 2,3-Dihydroxy-2-cyclopentenon.

Die Reduktone sind erfindungsgemäß bevorzugt in einer Menge von 0,1 Gew.-% bis 20 Gew.-%, insbesondere von 0,5 Gew.-% bis 10 Gew.-%, jeweils bezogen auf das Gewicht des anwendungsbereiten Mittels, in dem erfindungsgemäßen Mittel enthalten.

Als Träger für das erfindungsgemäße Mittel eignen sich bevorzugt flüssige Medien, in denen das erfindungsgemäße Sulfinsäurederivat bevorzugt löslich ist, wie beispielsweise Wasser oder organische Lösemittel. Es ist erfindungsgemäß bevorzugt, wenn der Träger ein kosmetischer Träger ist.

Als kosmetische Träger eignen sich besonders Cremes, Emulsionen, Gele oder auch tensidhaltige schäumende Lösungen, wie beispielsweise Shampoos, Schaumaerosole oder andere Zubereitungen, die insbesondere für die Anwendung auf dem Haar geeignet sind. Es ist aber auch denkbar, die Inhaltsstoffe in eine pulverförmige oder auch tablettenförmige Formulierung zu integrieren, welche vor der Anwendung in Wasser gelöst wird. Die kosmetischen Träger können insbesondere wässrig oder wässrig-alkoholisch sein. Ein wässriger kosmetischer Träger enthält mindestens 50 Gew.-% Wasser. Unter wässrig-alkoholischen kosmetischen Trägern sind im Sinne der vorliegenden Erfindung wässrige Lösungen enthaltend 3 bis 70 Gew.-% eines C₁-C₄-Alkohols, insbesondere Ethanol bzw. Isopropanol, zu verstehen. Weitere alkoholische Lösemittel, sind beispielsweise Methoxybutanol, Benzylalkohol, 2-Phenoxyethanol, Ethyldiglykol oder 1,2-Propylenglykol. In einer bevorzugten Ausführungsform enthält das erfindungsgemäße Mittel zusätzlich als Lösemittel mindestens einen (C₂ bis C₆)-Alkylmonoalkohol und/oder ein (C₂ bis C₆)-Alkandiol, insbesondere Ethanol, Isopropanol und/oder 1,2-Propylenglykol.

Das erfindungsgemäße Mittel besitzt bevorzugt einen pH-Wert von pH 1 bis pH 9, insbesondere von pH 1,5 bis pH 6.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Ein zweiter Gegenstand der Erfindung ist die Verwendung des Mittels gemäß erstem Erfindungsgegenstand zur Entfärbung von Substraten, die mit natürlichen und/oder synthetischen Farbstoffen eingefärbt sind, wobei die Substrate natürliche Fasern, ausgewählt aus keratinhaltigen Fasern, enthalten.

Das Substrat enthält natürliche Fasern. Die natürlichen Fasern werden ausgewählt aus keratinhaltigen Fasern, insbesondere Wolle oder tierischen oder menschlichen Haaren, ganz besonders bevorzugt aus menschlichen Haaren.

Die zu entfärbenden Substrate sind bevorzugt mit Oxidationsfarbstoffen und/oder direktziehenden Farbstoffen, als Vertreter der synthetischen Farbstoffe, gefärbt. Als zur Färbung des Substrats verwendete Entwicklerkomponenten können prinzipiell nachfolgende Entwicklerkomponenten dienen.

Erfindungsgemäß ganz besonders bevorzugte p-Phenylendiaminderivate sind p-Phenylendiamin, p-Toluylendiamin, 2-(β-Hydroxyethyl)-p-phenylendiamin, 2-(α,β-Dihydroxyethyl)-p-phenylendiamin und N,N-Bis-(β-hydroxyethyl)-p-phenylendiamin.

Es kann erfindungsgemäß weiterhin zur Färbung des Substrats bevorzugt sein, als Entwicklerkomponente Verbindungen einzusetzen, die mindestens zwei aromatische Kerne enthalten, die mit Amino- und/oder Hydroxylgruppen substituiert sind. Ganz besonders bevorzugte zweikernige Entwicklerkomponenten sind N,N'-Bis-(β-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan und 1,10-Bis-(2,5-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze zur Färbung des zu entfärbenden Substarts einzusetzen. Ganz besonders bevorzugte Verbindungen sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise den Pyridin-, Pyrimidin-, Pyrazol-, Pyrazol-PyrimidinDerivaten und ihren physiologisch verträglichen Salzen. Bevorzugte Pyridin-Derivate sind insbesondere die Verbindungen, wie 2,5-Diamino-pyridin, 2-(4-Methoxyphenyl)-amino-3-aminopyridin, 2,3-Diamino-6-methoxy-pyridin, 2-(β-Methoxyethyl)-amino-3-amino-6-methoxy-pyridin und 3,4-Diamino-pyridin. Bevorzugte Pyrimidin-Derivate sind insbesondere Verbindungen, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.
Bevorzugte Pyrazol-Derivate sind insbesondere Verbindungen, wie 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(β-aminoethyl)-amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4-(β-hydroxyethyl)-amino-1-methylpyrazol.

In einer weiteren bevorzugten Ausführungsform wurden die zu entfärbenden Substrate mit einem oxidativen Färbemittel gefärbt, welches neben mindestens einer Entwicklerkomponente zusätzlich mindestens eine Kupplerkomponente enthält. Erfindungsgemäß bevorzugte Kupplerkomponenten sind
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxy-ethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-[3-Morpholin-4-ylphenyl)amino]ethanol, 3-Amino-4-(2-methoxyethoxy)-5-methylphenylamin und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Amino-benzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol
sowie deren physiologisch verträglichen Salze.

Erfindungsgemäß besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Zusätzlich können die zu entfärbenden Substrate mit Vorstufen naturanaloger Farbstoffe bevorzugt unter Zuhilfenahme solcher Indole und Indoline gefärbt sein, die bevorzugt mindestens zwei Hydroxyoder Aminogruppen, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin. Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol. Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Das zu entfärbende Substrat kann ebenso mit direktziehenden Farbstoffen eingefärbt worden sein. Als direktziehende Farbstoffe kommen dabei insbesondere Nitrophenylendiamine, Nitroaminophenole, Azofarbstoffe, Anthrachinone oder Indophenole in Frage. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner können die zu entfärbenden Substrate bevorzugt mit einem kationischen direktziehenden Farbstoff gefärbt sein. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Weiterhin können die zu entfärbenden Substrate auch mit in der Natur vorkommenden, natürlichen Farbstoffen, wie sie beispielsweise in Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten sind, gefärbt sein.

Ein dritter Gegenstand der Erfindung ist ein Verfahren zur reduktiven Entfärbung von mit natürlichen und/oder synthetischen Farbstoffen eingefärbten Substraten, wobei die Substrate natürliche Fasern, ausgewählt aus keratinhaltigen Fasern, enthalten, in dem ein Mittel des ersten Erfindungsgegenstandes auf das eingefärbte Substrat aufgetragen wird und nach einer Einwirkzeit wieder abgespült wird.

Die Einwirkzeit beträgt bevorzugt 1 bis 60 Minuten, bevorzugt 5 bis 30 Minuten. Die Einwirkung des erfindungsgemäßen Mittels kann nicht nur bei Raumtemperatur, sondern bevorzugt in einem Temperaturbereich von 15 bis 60 °C, insbesondere von 25 bis 60 °C erfolgen.

Nach Ablauf der Einwirkzeit werden die Haare ausgespült, wobei bevorzugt ein tensidhaltiges Mittel, wie beispielsweise ein Reinigungsmittel oder ein Shampoo, Anwendung findet. Gegebenenfalls kann das Substrat mehrfach ausgespült, bzw. mehrfach mit dem tensidhaltigen Mittel behandelt werden.

Das tensidhaltige Mittel zur Spülung bzw. Nachbehandlung enthält bevorzugt in einem Träger mindestens ein Tensid, ausgewählt aus anionischen, zwitterionischen, ampholytischen, nichtionischen oder kationischen Tensiden, insbesondere aus nichtionischen Tensiden.

Als anionische Tenside eignen sich in den erfindungsgemäßen tensidhaltigen Mitteln zur Spülung bzw. Nachbehandlung alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen tensidhaltigen Mitteln zur Spülung bzw. Nachbehandlung verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid^{®}S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex^{®} vertriebenen Methyl-hydroxyalkyldialkoyloxyalkylammoniummethosulfate.

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Die Tenside werden bevorzugt in Konzentrationen von 0,5 bis 30 Gew.-%, bezogen auf das Gewicht der Zusammensetzung, in dem tensidhaltigen Mittel zur Spülung bzw. Nachbehandlung eingesetzt.

Zusätzlich besitzt das tensidhaltige Mittel zur Spülung bzw. Nachbehandlung einen pH-Wert von kleiner pH 7. Dieser pH-Wert wird bevorzugt durch den Zusatz eines Puffers während des Spül- bzw. Nachbehandlungsschritts konstant gehalten. Erfindungsgemäß bevorzugt geeignete Puffersysteme sind der Phosphatpuffer (PO₄³⁻ / HPO₄²⁻ / H₂PO₄⁻/ H₃PO₄), Essigsäure/Acetat-Puffer, Citronensäure/Citrat-Puffer, KCl / HCl-Puffer, Monophthalat/HCl-Puffer oder Monophthalat/NaOH-Puffer.

Es kann vorteilhaft sein, das Substrat anschließend mit einer oxidationsmittelhaltigen Zusammensetzung nachzubehandeln. Bevorzugt wird Wasserstoffperoxid als Oxidationsmittel, bevorzugt in Konzentrationen von 0,5 bis 6 Gew.-%, eingesetzt. Die Einwirkzeit beträgt bevorzugt 1 bis 30 Minuten, besonders bevorzugt 1 bis 10 Minuten. Nach Ablauf der Einwirkzeit wird die Oxidationsmittelhaltige Zusammensetzug ausgespült.

Als bevorzugte eingefärbte Substrate eignen sich diejenigen, die im zweiten Erfindungsgegenstand definiert wurden.

Als bevorzugte natürliche oder synthetische Farbstoffe wurden bevorzugt diejenigen Farbstoffe verwendet, wir sie im zweiten Erfindungsgegenstand definiert wurden.

Der Erfindungsgegenstand soll exemplarisch anhand der folgenden Ausführungen erläutert werden.

### Beispiele

Es wurden die erfindungsgemäßen Entfärbemittel gemäß Tabelle 1 bereitgestellt. Dabei wurden folgende Rohstoffe verwendet:

| Rohstoff: | Inhalt | Hersteller: |
|---|---|---|
| Brij^{®} 30 | Polyethylenglykolmonolaurylether, Dodecanol ethoxyliert mit ca. 4 Moläquivalent | Uniqema |
| | Ethylenoxid (INCI-Bezeichung: Laureth-4) | |
| Eumulgin^{®} B 2 | Ceteareth-20 | Cognis |
| Eumulgin^{®} B 3 | Ceteareth-30 | Cognis |
| Genapol^{®} UD 088 | C₁₁-Alkohol ethoxyliert mit ca. 8 Moläquivalent Ethylenoxid | Clariant |
| Brüggolit^{®} FF7 | enthält ca. 65 % 2-Hydroxy-2-sulfinoessigsäure Natriumsalz | Brüggemann |

Die Rohstoffe wurden nach und nach in 75 Gew.-% Wasser unter Rühren gemischt. Anschließend wurde der pH-Wert eingestellt und ggf. mit Wasser auf 100 Gew.-% aufgefüllt.

**Tabelle 1: Entfärbemittel**

| Rohstoff | E1 | E2 | E3 | E4 | E5 |
|---|---|---|---|---|---|
| | [Gew.-%] | [Gew.-%] | [Gew.-%] | [Gew.-%] | [Gew.-%] |
| Brüggolit^{®} FF7 | 10,0 | 10,0 | 10,0 | 10,0 | 10,0 |
| Brij^{®} 30 | 3,0 | 10,0 | - | - | - |
| Eumulgin^{®} B2 | - | - | 3,0 | - | - |
| Eumulgin^{®} B3 | - | - | - | 3,0 | - |
| Genapol^{®} UD 088 | - | - | - | - | 3,0 |
| H₃PO₄ | ad pH 1,5 | ad pH 1,5 | ad pH 1,5 | ad pH 1,5 | ad pH 1,5 |
| Wasser | ad 100 | ad 100 | ad 100 | ad 100 | ad 100 |

### Verfahren 1:

Folgende Prozedur wurde für alle Entfärbemittel der Tabelle 1 durchgeführt:
Eine Haarsträhne (Naturhaar, Fa. Kerling) wurde mit einer handelsüblichen oxidativen Haarfarbe (Igora^{®} Royal (Schwarzkopf), Färbecreme Naturton im Gewichtsverhältnis 1 zu 1 mit dem Entwickler Oxigenta^{®} (6 Gew.-% Wasserstoffperoxid, Fa. Schwarzkopf) über eine Einwirkzeit von 30 Minuten bei Raumtemperatur gefärbt, gespült und für 24 Stunden gelagert. Danach wurde die Strähne in 30 mL eines Entfärbemittels der Tabelle 1 über eine Einwirkzeit von 30 Minuten bei Raumtemperatur getaucht, anschließend für 3 Minuten unter fließendem Wasser gründlich gespült und an der Luft getrocknet.

Alle Haarsträhnen zeigten nach der Entfärbeprozedur eine hervorragende Aufhellung und eine geringe Haarschädigung. Die Haarsträhnen erfuhren auch nach 2 Wochen Lagerung keine Nachdunkelung.

### Verfahren 2:

Folgende Prozedur wurde für alle Entfärbemittel der Tabelle 1 durchgeführt:
Es wurde eine mit der oxidativen Haarfarbe gemäß Versuch 1 gefärbte Haarsträhne (Naturhaar, Fa. Kerling) in 30 mL eines der Entfärbemittel der Tabelle 1 über eine Einwirkzeit von 30 Minuten bei Raumtemperatur getaucht. Abweichend zum Versuch 1 wurde die Strähne jedoch anschließend für 2 Minuten in 50 mL eines der tensidhaltigen Mittel zur Spülung gemäß Tabelle 2 gewaschen, für weniger als 1 Minute unter fließendem Wasser gespült und an der Luft getrocknet.

**Tabelle 2: tensidhaltige Mittel zur Spülung**

| Rohstoff | B1 | B2 | B3 | B4 |
|---|---|---|---|---|
| | [Gew.-%] | [Gew.-%] | [Gew.-%] | [Gew.-%] |
| KH₂PO₄ | 5,0 | 5,0 | 5,0 | 5,0 |
| Brij^{®} 30 | 10,0 | - | - | - |
| Eumulgin^{®} B2 | - | 10,0 | - | - |
| Eumulgin^{®} B3 | - | - | 10,0 | - |
| Genapol^{®} UD 088 | - | - | - | 10,0 |
| H₃PO₄ | ad pH 2 | ad pH 2 | ad pH 2 | ad pH |
| Wasser | ad 100 | ad 100 | ad 100 | ad 100 |

Alle Haarsträhnen zeigten eine verbesserte Aufhellung im Vergleich zu einer Haarsträhne aus Verfahren 1, die lediglich mit Wasser gespült wurde. Die Haarschädigung ist gering. Die Haarsträhnen erfuhren auch nach 2 Wochen Lagerung keine Nachdunkelung.

## Patentansprüche

1. Mittel zum reduktiven Farbabzug keratinischer Fasern, enthaltend in einem Träger mindestens ein Sulfinsäurederivat der Formel (I) 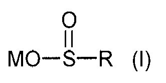 worin
M steht für ein Wasserstoffatom oder ein Äquivalent eines ein- oder mehrwertigen Kations,
R für einen Rest gemäß einer der Formeln (II) bis (III) steht, 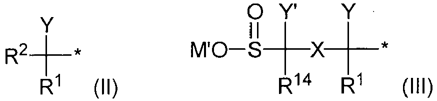 worin bedeuten
Y und Y' unabhängig voneinander eine Hydroxygruppe, eine -NH₂ Gruppe oder eine Gruppe -NR³R⁴, wobei R³ und R⁴ unabhängig voneinander für eine (C₁ bis C₆)-Alkylgruppe, eine (C₂ bis C₆)-Alkenylgruppe, eine (C₁ bis C₆)-Hydroxyalkylgruppe, eine (C₂ bis C₆)-Polyhydroxyalkylgruppe, eine Arylgruppe oder eine Aryl-(C₁ bis C₆)-alkylgruppe stehen,
M' unabhängig von M die unter M aufgeführten Merkmale,
X eine direkte Bindung oder einen organischen Rest mit zwei freien Valenzen,
R¹ und R¹⁴ unabhängig voneinander ein Wasserstoffatom, eine (C₁ bis C₆)-Alkylgruppe oder eine Carboxyalkylgruppe -(CH₂)ₘ-COOM^{II}, in der M^{II} für ein Wasserstoffatom oder für ein Äquivalent eines ein- oder mehrwertigen Kations steht und m die Zahl 0, 1 oder 2 bedeutet,
R² ein Wasserstoffatom, eine (C₁ bis C₆)-Alkylgruppe, einen Carboxyalkylrest -(CH₂)ₙ-COOM^{III} mit M^{III} = Wasserstoffatom oder ein Äquivalent eines ein- oder mehrwertigen Kations und n eine ganze Zahl 0, 1, 2, 3, 4, 5 oder 6, einen (C₁ bis C₆)-Alkyloxycarbonylrest, eine N,N,N-Tri[(C₁ bis C₆)-alkyl]ammonium-(C₁ bis C₆)-alkylgruppe eine Carboxy-(C₂ bis C₆)-alkenylgruppe, eine Cyano-(C₁ bis C₆)-alkylgruppe oder eine (C₁ bis C₆)-Alkoxycarbonyl-(C₁ bis C₆)-alkylgruppe, oder R² zusammen mit R¹ und dem Restmolekül einen aliphatischen 5-, 6-, oder 7-gliedrigen Ring bildet, welcher mindestens ein kationisches, quaterniertes Stickstoffatom als Heteroatom enthält, wobei die kationische Ladung gegebenenfalls durch ein Äquivalent eines ein- oder
mehrwertigen Anions kompensiert wird, oder einen aliphatischen oder aromatischen Heterozyklus, der substituiert sein kann, oder
einen Rest gemäß Formel (IV) 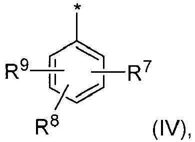 worin
R⁷ eine Carboxygruppe, eine Sulfonsäuregruppe, eine (C₁ bis C₆)-Alkoxycarbonylgruppe, eine Sulfonamidgruppe, eine Cyanogruppe, eine Nitrogruppe, eine Carboxy-(C₁ bis C₆)-alkylgruppe, eine Carboxy-(C₁ bis C₆)-alkoxygruppe oder eine Gruppe -N⁺R^{I}R^{II}R^{III}, mit R^{I}, R^{II} und R^{III} stehen unabhängig voneinander für eine (C₁ bis C₆)-Alkylgruppe, eine (C₂ bis C₆)-Alkenylgruppe, eine Aryl(C₁ bis C₆)-alkylgruppe oder eine (C₁ bis C₆)-Hydroxyalkylgruppe bedeutet,
R⁸ und R⁹ unabhängig voneinander ein Wasserstoffatom, eine (C₁ bis C₆)-Alkylgruppe, eine (C₂ bis C₆)-Alkenylgruppe, eine (C₁ bis C₆)-Hydroxyalkylgruppe, eine (C₂ bis C₆)-Polyhydroxyalkylgruppe, eine (C₁ bis C₆)-Alkoxygruppe, eine Hydroxygruppe, eine Aminogruppe, eine Carboxygruppe, eine Nitrogruppe, eine Cyanogruppe oder ein Halogenatom bedeuten,
mit der Maßgabe, dass in Formel (II) R¹ und R² nicht gleichzeitig für ein Wasserstoffatom stehen,
in Kombination mit mindestens einem nichtionischen Tensid, wobei das nichtionische Tensid ausgewählt wird aus mindestens einem Vertreter der Gruppe, die gebildet wird, aus Anlagerungsprodukten von 2 bis 30 Mol Ethylenoxid und/oder 0 bis 5 Mol Propylenoxid an lineare Fettalkohole mit 8 bis 22 C-Atomen.

2. Mittel nach Anspruch 1, **dadurch gekennzeichnet, daß** die Reste Y und Y' der Formel (I) unabhängig voneinander eine Hydroxygruppe oder eine Gruppe -NH₂ bedeuten.

3. Mittel nach einem der Ansprüche 1 bis 2, **dadurch gekennzeichnet, daß** der Rest R für eine Gruppe der Formel (II) steht.

4. Mittel nach einem der Ansprüche 1 bis 3, **dadurch gekennzeichnet, daß** es das Sulfinsäurederivat in einer Menge von 0,01 bis 20 Gew.-%, besonders bevorzugt von 1 bis 20 Gew.-%, jeweils bezogen auf das Gewicht des Mittels, enthält.

5. Mittel nach einem der Ansprüche 1 bis 4, **dadurch gekennzeichnet, daß** es die nichtionischen Tenside in einer Menge von 0,1 bis 25 Gew.-%, insbesondere 0,5 bis 15 Gew.-%, jeweils bezogen auf das gesamte Mittel, enthält.

6. Mittel nach einem der Ansprüche 1 bis 5, **dadurch gekennzeichnet, daß** das Gewichtsverhältnis der Sulfinsäurederivate der Formel (I) zu den nichtionischen Tensiden von 1 zu 2000 bis 10 zu 1, besonders bevorzugt von 1 zu 200 bis 2,5 zu 1, ganz besonders bevorzugt von 1 zu 50 bis 1 zu 1, beträgt.

7. Mittel nach einem der Ansprüche 1 bis 6, **dadurch gekennzeichnet, daß** es zusätzlich mindestens eine Verbindung, ausgewählt aus den Aldehyden oder den Ketonen, enthält.

8. Mittel nach einem der Ansprüche 1 bis 7, **dadurch gekennzeichnet, daß** es einen pH-Wert von pH 1 bis pH 9, insbesondere von pH 1,5 bis pH 6, besitzt.

9. Verwendung eines Mittels gemäß einem der Ansprüche 1 bis 8 zur Entfärbung von Substraten, die mit natürlichen und/oder synthetischen Farbstoffen eingefärbt sind, wobei die Substrate natürliche Fasern, ausgewählt aus keratinhaltigen Fasern, enthalten.

10. Verwendung nach Anspruch 9, **dadurch gekennzeichnet, daß** die zu entfärbenden Substrate mit Oxidationsfarbstoffen und/oder direktziehenden Farbstoffen gefärbt sind.

11. Verfahren zur reduktiven Entfärbung von mit natürlichen und/oder synthetischen Farbstoffen eingefärbten Substraten, wobei die Substrate natürliche Fasern, ausgewählt aus keratinhaltigen Fasern, enthalten, **dadurch gekennzeichnet, daß** ein Mittel gemäß einem der Ansprüche 1 bis 8 auf das eingefärbte Substrat aufgetragen wird und nach einer Einwirkzeit wieder abgespült wird.

12. Verfahren nach Anspruch 11, **dadurch gekennzeichnet, daß** die zu entfärbenden Substrate mit Oxidationsfarbstoffen und/oder direktziehenden Farbstoffen eingefärbt sind.

## Claims

1. An agent for reductive decolorization of keratin fibers, containing in a carrier at least one sulfinic acid derivative of formula (I) 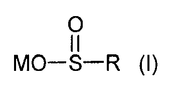 where
M represents a hydrogen atom or an equivalent of a monovalent or polyvalent cation,
R represents a functional group according to one of formulae (II) to (III), 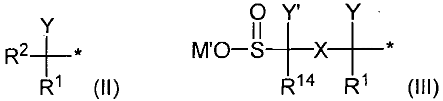 where
Y and Y' denote, independently one another, a hydroxy group, an -NH₂ group or a group -NR³R⁴, in which R³ and R⁴ represent, independently of one another, a (C₁ to C₆) alkyl group, a (C₂ to C₆) alkenyl group, a (C₁ to C₆) hydroxyalkyl group, a (C₂ to C₆) polyhydroxyalkyl group, an aryl group, or an aryl (C₁ to C₆) alkyl group,
M' denotes, independently of M, the features specified for M,
X denotes a direct bond or an organic functional group having two free valencies,
R¹ and R¹⁴ denote, independently of one another, a hydrogen atom, a (C₁ to C₆) alkyl group or a carboxyalkyl group -(CH₂)ₘ-COOM^{II}, in which M^{II} represents a hydrogen atom or an equivalent of a monovalent or polyvalent cation and m denotes the number 0, 1 or 2,
R² denotes a hydrogen atom, a (C₁ to C₆) alkyl group, a carboxyalkyl functional group -(CH₂)ₙ-COOM^{III} where M^{III} = a hydrogen atom or an equivalent of a monovalent or polyvalent cation and n = an integer 0, 1, 2, 3, 4, 5 or 6,
a (C₁ to C₆) alkyloxycarbonyl functional group, an N,N,N-tri[(C₁ to C₆) alkyl]ammonium (C₁ to C₆) alkyl group, a carboxy (C₂ to C₆) alkenyl group, a cyano (C₁ to C₆) alkyl group, or a (C₁ to C₆) alkoxycarbonyl (C₁ to C₆) alkyl group, or
R², together with R¹ and the remainder of the molecule, forms an aliphatic 5-member, 6-member or 7-member ring that contains at least one cationic, quaternized nitrogen atom as a heteroatom, wherein the cationic charge is optionally compensated for by an equivalent of a monovalent or polyvalent anion, or
an aliphatic or aromatic heterocycle that can be substituted, or a functional group of formula (IV) 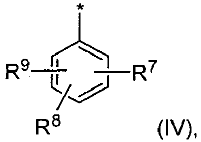 where
R⁷ denotes a carboxy group, a sulfonic acid group, a (C₁ to C₆) alkoxycarbonyl group, a sulfonamide group, a cyano group, a nitro group, a carboxy (C₁ to C₆) alkyl group, a carboxy (C₁ to C₆) alkoxy group, or a group -N⁺R^{I}R^{II}R^{III} where R^{I}, R^{II} and R^{III} represent, independently of one another, a (C₁ to C₆) alkyl group, a (C₂ to C₆) alkenyl group, an aryl (C₁ to C₆) alkyl group, or a (C₁ to C₆) hydroxyalkyl group,
R₈ and R₉ denote, independently of one another, a hydrogen atom, a (C₁ to C₆) alkyl group, a (C₂ to C₆) alkenyl group, a (C₁ to C₆) hydroxy alkyl group, a (C₂ to C₆) polyhydroxyalkyl group, a (C₁ to C₆) alkoxy group, a hydroxy group, an amino group, a carboxy group, a nitro group, a cyano group, or a halogen atom,
with the proviso that, in formula (II), R¹ and R² do not simultaneously represent a hydrogen atom,
in combination with at least one non-ionic surfactant, wherein the non-ionic surfactant is selected from at least one representative of the group that is formed by products of addition of from 2 to 30 mol ethylene oxide and/or from 0 to 5 mol propylene oxide to linear fatty alcohols having 8 to 22 C atoms.

2. The agent according to claim 1, **characterized in that** the functional groups Y and Y' of formula (I) denote, independently of one another, a hydroxy group or a group -NH₂.

3. The agent according to one of claims 1 to 2, **characterized in that** the functional group R represents a group of formula (II).

4. The agent according to one of claims 1 to 3, **characterized in that** it contains the sulfinic acid derivative in an amount of from 0.01 to 20 wt.%, particularly preferably from 1 to 20 wt.%, in each case based on the weight of the agent.

5. The agent according to one of claims 1 to 4, **characterized in that** it contains the non-ionic surfactants in an amount of from 0.1 to 25 wt.%, in particular from 0.5 to 15 wt.%, in each case based on the total agent.

6. The agent according to one of claims 1 to 5, **characterized in that** the weight ratio of the sulfinic derivatives of formula (I) to the non-ionic surfactants is from 1:2000 to 10:1, particularly preferably from 1:200 to 2.5:1, most particularly preferably from 1:50 to 1:1.

7. The agent according to one of claims 1 to 6, **characterized in that** it additionally contains at least one compound selected from the aldehydes or the ketones.

8. The agent according to one of claims 1 to 7, **characterized in that** it has a pH value of from pH 1 to pH 9, in particular from pH 1.5 to pH 6.

9. The use of an agent according to one of claims 1 to 8 for removing color from substrates that have been dyed using natural and/or synthetic dyes, wherein the substrates contain natural fibers selected from keratin-containing fibers.

10. The use according to claim 9, **characterized in that** the substrates from which color is to be removed have been dyed using oxidation dyes and/or substantive dyes.

11. A method for reductive removal of color from substrates that have been dyed using natural and/or synthetic dyes, the substrates containing natural fibers selected from keratin-containing fibers, **characterized in that** an agent according to one of claims 1 to 8 is applied to the dyed substrate and is rinsed off again after a contact time.

12. The method according to claim 11, **characterized in that** the substrates from which color is to be removed have been dyed using oxidation dyes and/or substantive dyes.

## Revendications

1. Agent pour l'élimination par réduction de la couleur de fibres kératiniques, contenant dans un support au moins un dérivé d'acide sulfinique de la formule (I) 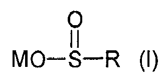 dans lequel
M représente un atome d'hydrogène ou un équivalent d'un cation mono- ou plurivalent,
R représente un résidu selon l'une des formules (II) à (III), 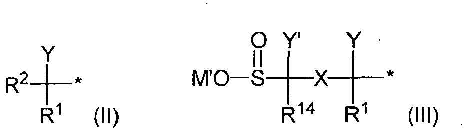 dans lesquelles
Y et Y' représentent indépendamment l'un de l'autre un groupe hydroxy, un groupe -NH₂ ou un groupe -NR³R⁴, dans lequel R³ et R⁴ représentent indépendamment l'un de l'autre un groupe alkyle (en C₁ à C₆), un groupe alcényle (en C₂ à C₆), un groupe hydroxyalkyle (en C₁ à C₆), un groupe polyhydroxyalkyle (en C₂ à C₆), un groupe aryle ou un groupe arylalkyle (en C₁ à C₆),
M' représente, indépendamment de M, les caractères énoncés sous M,
X représente une liaison directe ou un résidu organique doté de deux valences libres,
R¹ et R¹⁴ représentent indépendamment l'un de l'autre un atome d'hydrogène, un groupe alkyle (en C₁ à C₆) ou un groupe carboxyalkyle -(CH₂)ₘ-COOM^{II}, dans lequel M^{II} représente un atome d'hydrogène ou un équivalent d'un cation mono- ou plurivalent et m représente les chiffres 0, 1 ou 2
R² représente un atome d'hydrogène, un groupe alkyle (en C₁ à C₆), un résidu carboxyalkyle -(CH₂)ₙ-COOM^{III} avec
M^{III} = atome d'hydrogène ou un équivalent d'un cation mono- ou plurivalent et n est un nombre entier 0, 1, 2, 3, 4, 5 ou 6,
un résidu alkyloxycarbonyle (en C₁ à C₆), un groupe alkyle (en C₁ à C₆) de N,N,N-trialkylammonium (en C₁ à C₆), un groupe carboxyalcényle (en C₂ à C₆), un groupe cyanoalkyle (en C₁ à C₆) ou un groupe (C₁ to C₆) alkoxycarbonylalkyle (en C₁ à C₆), ou
R² avec R¹ et la molécule résiduelle forment un anneau aliphatique à 5, 6 ou 7 branches, lequel contient au moins un atome d'azote cationique quaternaire en tant qu'hétéroatome, dans lequel la charge cationique est le cas échéant compensée par un équivalent d'un anion mono- ou plurivalent, ou
un hétérocyle aliphatique ou aromatique qui peut être substitué, ou un résidu selon la formule (IV) 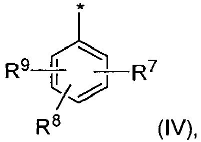 dans laquelle
R⁷ représente un groupe carboxy, un groupe d'acide sulfonique, un groupe alkoxycarbonyle (en C₁ à C₆), un groupe sulfonamide, un groupe cyano, un groupe nitro, un groupe carboxyalkyle (en C₁ à C₆), un groupe carboxyalkoxy (en C₁ à C₆) ou un groupe -N⁺R^{I}R^{II}R^{III}, avec R^{I}, R^{II} et R^{III} représentant indépendamment l'un de l'autre un groupe alkyle (en C₁ à C₆), un groupe alcényle (en C₂ à C₆), un groupe arylalkyle (en C₁ à C₆) ou un groupe hydroxyalkyle (en C₁ à C₆),
R⁸ et R⁹ représentent indépendamment l'un de l'autre un atome d'hydrogène, un groupe alkyle (en C₁ à C₆), un groupe alcényle (en C₂ à C₆), un groupe hydroxyalkyle (en C₁ à C₆), un groupe polyhydroxyalkyle (en C₂ à C₆), un groupe alkoxy (en C₁ à C₆), un groupe hydroxy, un groupe amino, un groupe carboxy, un groupe nitro, un groupe cyano ou un atome d'halogène,
à la condition que R¹ et R² ne représentent pas simultanément un atome d'hydrogène dans la formule (II),
en combinaison avec au moins un tensioactif non-ionique, dans lequel le tensioactif non-ionique est sélectionné parmi au moins un représentant du groupe constitué des produits d'addition de 2 à 30 mol d'oxyde d'éthylène et/ou de 0 à 5 mol d'oxyde de propylène sur des alcools gras linéaires dotés de 8 à 22 atomes C.

2. Agent selon la revendication 1, **caractérisé en ce que** les résidus Y et Y' de la formule (I) représentent indépendamment l'un de l'autre un groupe hydroxy ou un groupe -NH₂.

3. Agent selon l'une quelconque des revendications 1 à 2, **caractérisé en ce que** le résidu R représente un groupe de la formule (II).

4. Agent selon l'une quelconque des revendications 1 à 3, **caractérisé en ce qu'**il contient le dérivé d'acide sulfinique dans une quantité de 0,01 à 20 % en poids, très préférablement de 1 à 20 % en poids, respectivement rapporté au poids de l'agent.

5. Agent selon l'une quelconque des revendications 1 à 4, **caractérisé en ce qu'**il contient les tensioactifs non-ioniques dans une quantité de 0,1 à 25 % en poids, notamment de 0,5 à 15 % en poids, respectivement rapporté à l'ensemble de l'agent.

6. Agent selon l'une quelconque des revendications 1 à 5, **caractérisé en ce que** le rapport de poids des dérivés d'acide sulfinique de la formule (I) sur les tensioactifs non-ioniques s'élève de 1 sur 2 000 à 10 sur 1, très préférablement de 1 sur 200 à 2,5 sur 1, encore plus préférablement de 1 sur 50 à 1 sur 1.

7. Agent selon l'une quelconque des revendications 1 à 6, **caractérisé en ce qu'**il contient en outre au moins un composé sélectionné parmi les aldéhydes ou les cétones.

8. Agent selon l'une quelconque des revendications 1 à 7, **caractérisé en ce qu'**il possède une valeur pH de pH 1 à pH 9, notamment de pH 1,5 à pH 6.

9. Utilisation d'un agent selon l'une quelconque des revendications 1 à 8 pour la décoloration de substrats qui sont colorés avec des colorants naturels et/ou de synthèse, dans lequel les substrats contiennent des fibres naturelles, sélectionnées parmi les fibres kératiniques.

10. Utilisation selon la revendication 9, **caractérisée en ce que** les substrats à décolorer sont colorés par des colorants par oxydation et/ou par des colorants à action directe.

11. Procédé pour la décoloration par réduction de substrats colorés par des colorants naturels et/ou de synthèse, dans lequel les substrats contiennent des fibres naturelles, sélectionnées parmi les fibres kératiniques, **caractérisé en ce qu'**un agent selon l'une quelconque des revendications 1 à 8 est appliqué sur le substrat coloré et de nouveau rincé après une durée d'action.

12. Procédé selon la revendication 11, **caractérisé en ce que** les substrats à décolorer sont colorés avec des colorants par oxydation et/ou par des colorants à action directe.
